(19) Europäisches Patentamt
European Patent Office
Office européen des brevets

(11) Numéro de publication: **0 039 780**
**B1**

(12) **FASCICULE DE BREVET EUROPEEN**

(45) Date de publication du fascicule du brevet: **10.04.85**

(21) Numéro de dépôt: **81102573.3**

(22) Date de dépôt: **06.04.81**

(51) Int. Cl.⁴: **C 07 D 473/06,** A 61 K 31/52
// C07D239/54, C07D239/62

(54) **Xanthines di- ou trisubstituées ayant des propriétés neuroleptiques, procédés de préparation et médicament les contenant.**

(30) Priorité: **02.05.80 CH 3431/80**

(43) Date de publication de la demande:
**18.11.81 Bulletin 81/46**

(45) Mention de la délivrance du brevet:
**10.04.85 Bulletin 85/15**

(84) Etats contractants désignés:
**BE CH DE FR IT LI NL SE**

(56) Documents cités:
**EP-A-0 019 165**
**DE-B-1 091 570**
**FR-A-2 346 353**
**GB-A- 701 242**
**GB-A- 759 174**
**GB-A- 766 754**
**GB-A- 982 079**
**US-A-2 673 848**

**Le dossier contient des informations techniques présentées postérieurement au dépôt de la demande et ne figurant pas dans le présent fascicule.**

(73) Titulaire: **SOCIETE DES PRODUITS NESTLE S.A.**
**Case postale 353**
**CH-1800 Vevey (CH)**

(72) Inventeur: **Philippossian, Georges**
**18, av. de Valmont**
**CH-1010 Lausanne (CH)**
Inventeur: **Enslen, Marc**
**Rue de Chamblon 25**
**CH-1400 Yverdon (CH)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 039 780 B1

**Description**

La présente invention concerne des xanthines pharmacologiquement actives, des procédés pour leur préparation et un médicament les contenant.

Les xanthines sont connues pour leur action excitante du système nerveux central. On peut citer par exemple la caféine ou la théophylline.

Certaines xanthines ont été décrites pour leur activité spasmolytique, par exemple dans la demande de brevet allemand No. 2.713.389 ou la demande de brevet européen No. 1735.

Le document EP—A—19165 décrit la 1-isopropyl- et la 1-isobutyl-3,7-diméthylxanthine comme ayant des propriétés sédatives.

Nous avons trouvé que des xanthines 1,3 et 1,3,8 substituées, contraitement aux xanthines psychostimulantes utilisées en thérapeutique, exercent une activité sédative ou anxiolytique sans qu'on observe d'effet secondaire aux doses efficaces (ED50) pour l'activité neuroleptique.

Certaines d'entre elles montrent une activité annexe diurétique, antiallergique, bronchodilatatrice ou antihistaminique à des doses minimales efficaces, c'est-à-dire produisant une réponse significative dans un screening de base, nettement supérieure aux doses efficaces (ED50) pour l'effet neuroleptique, c'est-à-dire que cette activité annexe ne déprécie en aucune manière la valeur de l'activité neuroleptique. Les composés selon l'invention sont les xanthines de formule générale

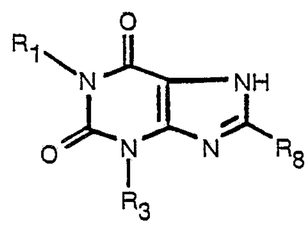

(I)

ou leurs sels physiologiquement acceptables, formule dans laquelle

$R_1$ est alkyle en $C_2$—$C_4$, isoalkyle en $C_3$—$C_4$, $CH_2$-(alcényle en $C_2$—$C_3$) ou $CH_2$-(isoalcényle en $C_3$),

$R_3$ est alkyle en $C_{3-5}$, isoalkyle en $C_3$—$C_5$, $CH_2$-(alcényle en $C_2$—$C_4$) ou $CH_2$-(isoalcényle en $C_3$—$C_4$),

$R_8$ est H, méthyle ou éthyle,

sauf que

1) lorsque $R_8$ est H, $R_1$ est allyle,

2) $R_1$ et $R_3$ ne sont pas simultanément butyle, allyle ou isobutyle et

3) lorsque $R_1$ est éthyle, $R_3$ est différent de 2-méthylbutyle.

Les composés de formule générale (I) préférés sont ceux pour lesquels $R_1$ est allyle, propyle ou isobutyle, $R_3$ est propyle, butyle ou isobutyle et $R_8$ est méthyle, les composés 1-allyl-3-butyl-8-méthyl-, 1-propyl-3-butyl-8-méthyl-, 1-allyl-3-isobutyl-8-méthyl-, 1,3-dipropyl-8-méthyl-, 1-propyl-3-isobutyl-8-méthyl- et 1-allyl-3-propyl-8-méthyl-xanthine étant particulièrement préférés.

Les composés pour lesquels $R_1$ est allyle, $R_3$ est butyle ou isobutyle et $R_8$ est H constituent également une catégorie préférée.

Par sels physiologiquement acceptables des composés de formule générale (I), on entend les sels que forment ces composés avec des bases pharmaceutiquement acceptables: il s'agit de sels dont les cations présentent une inocuité vis-à-vis des organismes animaux et ne provoquent pas d'effet secondaire propre aux doses thérapeutiques. On peut citer les sels de métaux alcalins tels que le sodium, le potassium, les sels d'ammonium et d'amines pharmaceutiquement acceptables connues de spécialiste. Ces sels peuvent être préparés par chauffage du composé de formule générale (I) en présence de la base appropriée avec ou sans solvant, de préférence suivi d'une recristallisation.

Les composés peuvent être préparés par l'un des procédés ci-après:

A. On fait réagir un uracile de formule

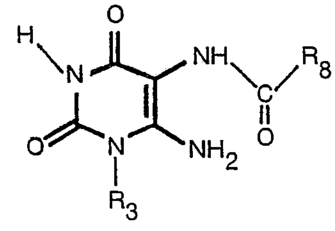

$(II_A)$

avec un agent alkylant de formule $R_1$—X où $R_1$, $R_3$ et $R_8$ sont définis comme dans la formule générale (I)

sauf que ni $R_1$ ni $R_3$ ne peuvent être isopropyle et où X est un halogène, de préférence le brome, ou mono- ou disulfate ou p-toluène sulfonate dans un solvant mutuel des reactifs tels que par exemple la diméthylformamide (DMF), le diméthylsulfoxyde (DMSO) ou l'hexaméthylphosphorotriamide (HMPT) à une température comprise entre 20 et 40°C et en présence d'un hydroxyde alcalin, par exemple l'hydroxyde de sodium sous forme solide. La réaction est conduite de préférence dans la DMF à 20°C selon le schéma réactionnel ci-après

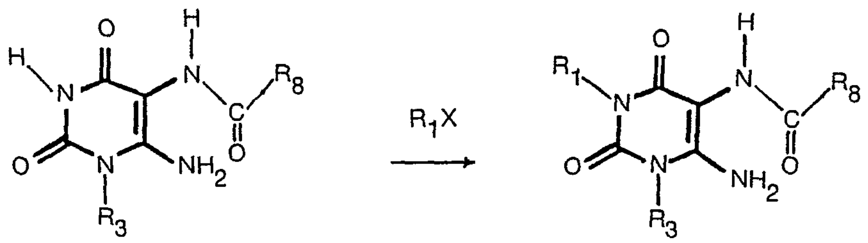


($III_A$)

On opère ensuite la cyclisation du produit de formule générale ($III_A$) dans une solution d'hydroxyde alcalin bouillant selon la schéma réactionnel ci-dessous:

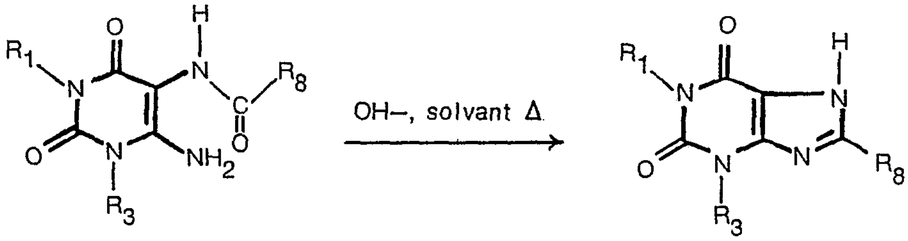


($I_A$)

Bien qu'il soit possible d'isoler le dérivé de formule générale ($III_A$), on préfère opérer directement la cyclisation sans isoler ou purifier celui-ci. A cet effet, on neutralise le milieu réactionnel et on évapore le solvant, puis on dissout le résidu dans une solution d'hydroxyde alcalin et on chauffe au reflux.

Les étapes précédentes de ce procédé conduisant à l'uracile de départ de formule générale ($II_A$) font appel à des méthodes traditionnelles, celle de Traube par exemple (Chem. Ber. *33*, 1371 et 3055, 1900), par réaction de l'urée

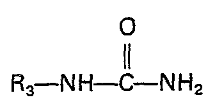

avec l'acide cyanoacétique dans l'anhydride acétique, évaporation de l'anhydride acétique et traitement du résidu par la soude, conduisant au 1-alkyl-6-aminouracile et transformation de ce dernier en composé de formule générale ($II_A$) suivant le schéma réactionnel suivant:

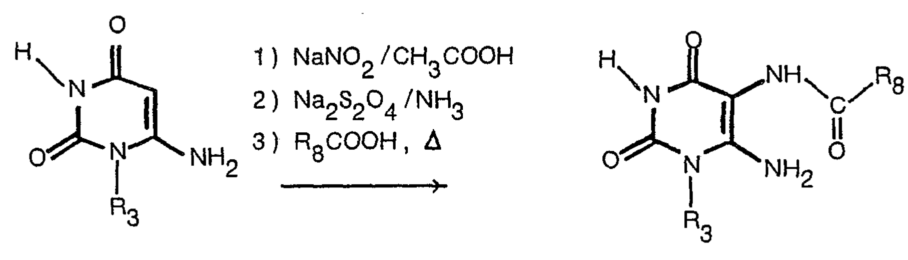


($II_A$)

B. En variante du procédé ci-dessus on peut effectuer l'hydrogénation d'un composé de formule générale (I) dans lequel ou moins 1 des substituants $R_1$ et $R_3$ est alcényle ou isoalcényle de façon à obtenir les composés saturés correspondants dont les deux mêmes groupes sont alkyle ou isoalkyle. On a en effet constaté que cette voie de synthèse est préférée dans les cas où on veut obtenir un composé de formule générale (I), où $R_1$ est propyle, butyle ou isobutyle et $R_3$ est alkyle différent de $R_1$, en particulier parce que l'étape d'alkylation conduisant au composé de formule générale ($III_B$) s'effectue avec de meilleurs rendements avec un agent alkylant du type

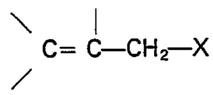

où X est défini comme précédemment.

On conduit l'hydrogénation catalytique du ou des 2 groupes alcényle ou isoalcényle dans un bon solvant des xanthines, tels que le méthanol, l'éthanol ou l'acétate d'éthyle en présence d'un catalyseur d'hydrogénation, tels que le Nickel de Raney, le Palladium sur charbon actif (Pd/C) ou l'oxyde de Platine. Généralement on préfère utiliser l'éthanol et le Pd/C. Bien qu'il soit possible de travailler sous pression et en chauffant, on préfère les conditions normales, c'est-à-dire T ambiante et pression atmosphérique.

C. Selon une autre variante qu'on préfère utiliser pour préparer les composés de formule générale (I) dans lesquels $R_1$ et $R_3$ sont identiques, mais différents de isopropyle, on part d'une urée identiquement substituée en 1 et en 3 qu'on traite par l'acide cyanoacétique en présence d'anhydride acétique, puis par la soude, selon la synthèse classique de Traube pour conduire au 1,3-dialkyl-6-aminouracile qu'on transforme ensuite en composé de formule générale ($III_C$) selon le schéma réactionnel ci-dessous, ce qui permet d'éviter l'étape d'alkylation:

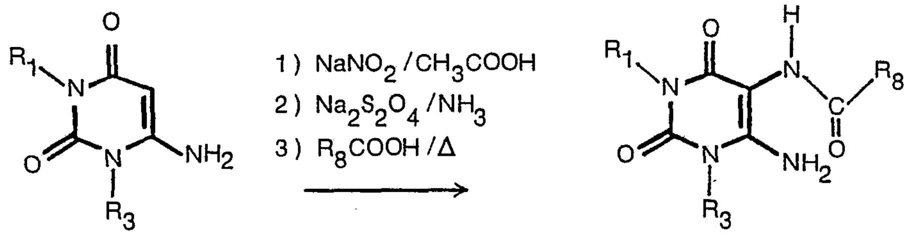


($III_C$)

Le composé de formule générale ($III_C$) ainsi obtenu est ensuite cyclisé comme indiqué ci-dessus en liaison avec le procédé A pour conduire au composé de formule générale ($I_C$) correspondant.

D. Un dernier procédé s'applique en particulier aux composés de formule générale (I) pour lesquels au moins l'un des groupes $R_1$ et $R_3$ est isopropyle. Celui-ci est analogue au procédé décrit par Goldner, Dietz et Carstens, par exemple dans Justus Liebigs Ann. Chem. *691*, 142, 1966.

On part d'une urée monosubstituée

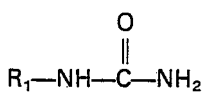

que l'on fait réagir avec le malonate de diéthyle dans l'éthanol en présence d'éthylate de sodium et on obtient un acide barbiturique 1-substitué qu'on fait réagir avec un agent de chloruration, par exemple l'oxychlorure de phosphore pour donner un 6-chlorouracile, le substituant $R_1$ se trouvant alors en position 3. Le 6-chlorouracile est ensuite traité avec un agent alkylant $R_3$—X pour donner un uracile 1-$R_3$,3-$R_1$-dialkylsubstitué. Ce dernier, en présence d'une amine de la forme $R_8$—$CH_2$—$NH_2$ donne le dérivé 6-amino, qui est nitrosé, par exemple par le nitrilé d'isopentyle en position 5, en l'uracile de formule générale (IV) ci-dessous. Ce dernier cyclise soit spontanément, soit en le chauffant, en une xanthine de formule générale ($I_D$) comme indiqué dans le schéma ci-dessous:

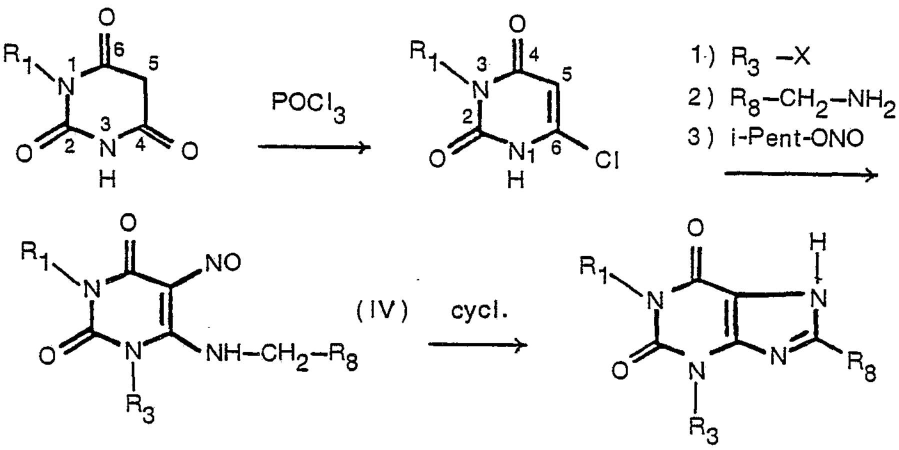


($I_D$)

L'invention concerne également une composition pharmaceutique contenant un composé de formule générale I en combinaison avec un support inerte pharmaceutiquement acceptable, saaf que l'exclusion 2) dans la formule générale I se lit: "$R_1$ et $R_3$ ne sont pas simultanement butyle ou allyle.".

Le médicament selon l'invention peut se présenter sous diverses formes pharmaceutiques contenant les exipients ou véhicules habituels tels que les comprimés, capsules, suppositoires, solutions, suspensions et être administrés par voie orale, sublinguale, rectale, sous-cutanée, intramusculaire, intraveineuse ou par inhalation à des doses comprises entre 0,0004 et 0,04 g par jour.

Les exemples suivants illustrent l'invention; dans ceux-ci les quantités sont pondérales sauf indication contraire:

Exemple 1

Synthèse de la 1-allyl-3-butyl-8-méthylxanthine

1) 1-butyl-6-aminouracile

On chauffe pendant 2 heures à 75—80°C 115 g (1 mole) de butylurée et 94 g (1,1 mole) d'acide cyanoacétique dans 200 ml d'anhydride acétique. On refroidit le mélange et on ajoute 500 ml d'éther. On récolte le précipité, on lave à l'éther et on sèche.

On suspend ce produit dans un mélange de 300 ml d'eau et de 150 ml d'éthanol. On chauffe à 85°C et on ajoute lentement 75 ml d'une solution aqueuse de NaOH à 10%. Le solide se solubilise et peu de temps après l'uracile se met à précipiter. L'addition terminée, on laisse encore réagir durant 30 min. On acidifie à pH 5 avec du HCl, on laisse refroidir et on filtre le précipité. On lave avec de l'eau glacée: 100 g de cristaux en poudre incolore.

2) 1-butyl-5-nitroso-6-aminouracile

Dans un réacteur muni d'un agitateur magnétique, on suspend 91,5 g (0.5 mole) de 1-butyl-6-aminouracile dans 1 litre d'eau. On ajoute à la suspension une solution de 38 g de nitrite de sodium dans 250 ml d'eau. On laisse ensuite couler goutte à goutte 65 ml d'acide acétique tout en agitant et on laisse sous agitation durant 18 heures à température ambiante. On refroidit dans un bain de glace et on filtre le précipité: 90 g de cristaux violets.

3) 1-butyl-5,6-diaminouracile

On suspend 84,8 g (0.4 mole) de 1-butyl-5-nitroso-6-aminouracile dans 440 ml d'une solution aqueuse concentrée à 50% d'hydroxyde d'ammonium. On chauffe cette suspension à 80°C. On ajoute par portions dans un laps de temps de 30 min et sous agitation 88 g (0,48 mole) de dithionite de sodium. On poursuit encore l'agitation 30 min à 80°C, puis 1 nuit à température ambiante. On refroidit dans de la glace, on filtre le précipité et on le lave avec un peu d'eau glacée: 62 g de cristaux en poudre très fine.

4) 1-butyl-5-acétylamino-6-aminouracile

Dans un réacteur muni d'un réfrigérant, on chauffe à reflux pendant 2 heures et sous agitation 59,4 g (0,3 mole) de 1-butyl-5,6-diaminouracile dans 240 ml d'acide acétique. On évapore l'acide acétique et on reprend le résidu par un peu d'éthanol, qu'on évapore. On répète cette opération jusqu'à obtention d'un résidu semicristallin, qu'on triture alors dans l'éther jusqu'à solidification complète. On filtre le solide et on le lave à l'éther: 72 g de fins cristaux légèrement jaunes.

5) 1-allyl-3-butyl-8-méthylxanthine

On dissout 13,2 g (0,055 mole) de 1-butyl-5-amino-6-acétylaminouracile dans 110 ml de diméthylformamide. On ajoute à cette solution et sous agitation 2,4 g (0,06 mole) de NaOH et 7,3 g (0,06 mole) de bromure d'allyle. La réaction se poursuit à température ambiante pendant 30 à 60 min.

On neutralise à pH 5 avec une solution de HCl concentré et on évapore la diméthylformamide. On dissout le résidu huileux dans 40 ml d'une solution de NaOH à 10% et on chauffe cette solution durant 2 heures à reflux. On refroidit à température ambiante, lave avec du dichlorométhane (2 × 10 ml) et ajuste à pH 5 avec une solution de HCl concentré. On extrait le précipité avec du dichlorométhane (3 × 20 ml), puis on sèche cette solution et on évapore: 6,5 g de cristaux colorés.

On décolore par traitement un charbon actif dans l'éthanol bouillant pendant 1 heure. On recristallise dans un mélange éthanol/eau 1/1: cristaux ouatés incolores. Point de fusion (PF): 170—171°C. Spectre de résonance magnétique nucléaire du carbone (C-RMN): voir tableau 2 ci-après.

Exemples 2—10

En utilisant le mode opératoire décrit dans l'exemple 1, on prépare les composés en utilisant les réactifs appropriés contenant les groupes $R_1$, $R_3$ et $R_8$, c'est-à-dire respectivement: l'urée monosubstituée dans l'étape 1, l'acide carboxylique dans l'étape 4 et l'agent alkylant dans l'étape 5, ainsi que l'indique le *tableau 1*. Dans le *tableau 2* figurent le solvant de recristallisation, les points de fusion et les valeurs de RMN du carbone.

TABLEAU 1

| Exemples | Composé | Urée (étape 1) | Acide (étape 4) | Agent alkylant (étape 5) |
|---|---|---|---|---|
| 2 | 1-allyl-3-butylxanthine | Butylurée | Formique | Bromure d'allyle |
| 3 | 1-allyl-3-isobutylxanthine | Isobutylurée | Formique | Bromure d'allyle |
| 4 | 1-allyl-3-isobutyl-8-méthylxanthine | Isobutylurée | Acétique | Bromure d'allyle |
| 5 | 1-allyl-3-butyl-8-éthylxanthine | Butylurée | Propionique | Bromure d'allyle |
| 6 | 1-allyl-3-propyl-8-méthylxanthine | Propylurée | Acétique | Bromure d'allyle |
| 7 | 1-allyl-3-isopentyl-8-méthylxanthine | Isopentylurée | Acétique | Bromure d'allyle |
| 8 | 1-ethyl-3-butyl-8-méthylxanthine | Butylurée | Acétique | Diéthylsulfate |
| 9 | 1,8-diéthyl-3-butylxanthine | Butylurée | Propionique | Diéthylsulfate |
| 10 | 1-butyl-3-allyl-8-méthylxanthine | Allylurée | Acétique | p-toluènesulfonate de butyle |

Exemple 11

Synthèse de la 1-propyl-3-butyl-8-méthylxanthine

Dans un appareil à hydrogéner, on met en solution 5 g de 1-allyl-3-butyl-8-méthylxanthine (exemple 1) dans 100 ml d'éthanol et on ajoute 500 mg de Pd 10%/C. On hydrogène à T ambiante jusqu'à l'arrêt de la consommation d'hydrogène (environ 1 heure). On filtre le mélange à chaud, on lave le prècipitè avec de l'éthanol et on évapore le solvant du filtrat: 5 g de cristaux incolores. On recristallise dans le méthanol. PF: 174—175°C. C-RMN (tableau 2).

Exemples 12 et 13

En utilisant le procédé de l'exemple 11, on prépare: la *1-propyl-3-isobutyl-8-méthylxanthine* à partir de la 1-allyl-3-isobutyl-8-méthylxanthine (exemple 4), et la *1-butyl-3-propyl-8-méthylxanthine* à partir de la 1-butyl-3-allyl-8-méthylxanthine (exemple 10).

Le point de fusion, le solvant de recristallisation et les valeurs de RMN du carbone de ces composés figurent dans le tableau 2 ci-après.

Exemple 14

Préparation de al 1,3-dipropyl-8-méthylxanthine

1) 1,3-dipropyl-6-aminouracile

On chauffe durant 2-heurs à 70°C 37,5 g (0,26 mole) de 1,3-dipropylurée et 22,5 g (0,26 mole) d'acide cyanoacétique dans 125 ml d'anhydride acétique. On évapore l'anhydride acétique: env. 90 g de résidu huileux.

On ajoute lentement à ce résidu 290 ml d'une solution aqueuse de NaOH à 10%, tout en agitant. Le mélange s'échauffe et l'huile se dissout. Sitôt après, il se forme un précipité. On laisse refroidir à T ambiante, on filtre le précipité, on le lave à l'eau et on le sèche: 55 g de poudre cristalline légèrement jaune.

2) 1,3-dipropyl-5-nitroso-6-aminouracile

On prépare ce composé de la même manière que dans l'exemple 1, étape 2.

3) 1,3-dipropyl-5,6-diaminouracile

On prépare ce composé selon la description de l'exemple 1.3.

4) 1,3-dipropyl-5-acétylamino-6-aminouracile

Ce composé est prépare selon la description de l'exemple 1.4.

5) 1,3-dipropyl-8-méthylxanthine

On chauffe durant 1 heure au reflux 15 g de 1,3-dipropyl-5-acétylamino-6-aminouracile (4) dans 150 ml d'une solution aqueuse de NaOH à 10%. On refroidit la solution et on l'acidifie avec du HCl concentré jusqu'à pH 2. On filtre le précipité formé, on le lave avec de l'eau et on le sèche: 13 g de cristaux colorés.

On décolore par traitement au charbon actif dans un mélange éthanol/eau 1/1 pendant 1 heure. On recristallise dans un mélange de même composition: cristaux ouatés incolores: PF: 202—203°C. C-RMN (tableau 2).

Exemple 15

Préparation de la 1-isopropyl-3-butyl-8-méthylxanthine

1) Acide 1-isopropylbarbiturique

On dissout 13,8 g (0,6 mole) de sodium dans 250 ml d'éthanol anhydre. On ajoute à cette solution 61,2 g (0,46 mole) de malonate de diéthyle et 47,3 g d'isopropylurée (0,46 mole). On chauffe ce mélange durant 7,5 heures à 120°C (température du bain). On laisse refroidir et on ajoute lentement une solution de HCl conc/$H_2O$ 1/4 jusqu'à dissolution du précipité formé durant la réaction. On évapore cette solution jusqu'à apparition d'un précipité et on abandonne à 0°C. On filtre le précipité, on le lave à l'eau froide et on le sèche: 75 g de cristaux incolores.

2) 3-isopropyl-6-chlorouracile

On laisse lentement couler sur 75 g (0,44 mole) d'acide 1-isopropyl-barbiturique 350 ml d'oxychlorure de phosphore contenant 15 ml d'eau. Une fois que la violente réaction exothermique s'est apaisée, on chauffe encore 1 heure au reflux. On chasse l'excès de $POCl_3$ par évaporation sous vide et on jette le résidu huileux dans de la glace. On obtient ainsi un précipité qu'on filtre, lave à l'eau glacée et sèche: 55 g de cristaux jaunâtres.

3 1-butyl-3-isopropyl-6-chlorouracile

A une solution de 19 g (0,10 mole) de 3-isopropyl-6-chlorouracile dans 200 ml de diméthylformamide, on ajoute 16,6 g (0,12 mole) de carbonate de potassium et 24 g (0,105 mole) de p-toluènesulfonate de butyle. On chauffe ce mélange durant 1 heure à 80°C. On évapore le solvant, on reprend le résidu dans de l'eau et on extrait avec du $CH_2Cl_2$. On sèche l'extrait sur du $Na_2SO_4$ et on évapore le solvant: 24 g de produit huileux jaune pâle.

4) 1-butyl-3-isopropyl-6-éthylaminouracile

A l'huile précédente (0,1 mole), on ajoute 100 ml d'éthanol et 100 ml d'une solution d'éthylamine à 70% dans l'eau. On chauffe le tout à reflux pendant 2 heures, puis on évapore à sec: 25 g de produit huileux jaune qu'on utilise sans purification pour la suite.

5) 1-isopropyl-3-butyl-8-méthylxanthine

L'huile précédente est dissoute dans 100 ml d'éthanol. On ajoute 30 gouttes d'un solution éthanolique de HCl, puis sous agitation 25 ml de nitrite d'isopentyle (addition lente, 15 min). On laisse réagir pendant 1 heure encore: la solution est violet foncé. On évapore la solution à sec, puis on dissout le résidu dans du NaOH dil. et on acidifie avec du HCl conc. jusqu'à pH 2. On abandonne à 0°C et on filtre le précipité formé. On dissout ce précipité dans du chloroforme et on le filtre sur une colonne contenant 50 g de gel de silice en éluant avec du chloroforme: 10 g de cristaux légèrement colorés. On recristallise 2 fois le produit dans un mélange d'éthanol et d'eau 1/1: fins cristaux incolores. PF: 172—173°C. C-RMN (tableau 2).

TABLEAU 2

| Exemples | PF (°C) | Solvant recristallis. | Valeurs C–RMN ($CDCl_3$; δ: ppm) Référence: TMS (tétraméthylsilane) |
|---|---|---|---|
| 1 | 170–171 | EtOH/$H_2O$ 1:1 | 13,8; 14,8; 20,0; 30,2; 43,8; 43,8; 106,7; 117,3; 132,4; 149,7; 150,7; 152,4; 155,6; |
| 2 | 149–150 | MeOH | 13,8; 20,0; 30,2; 43,7; 43,9; 107,0; 117,5; 132,2; 140,6; 149,0; 157,7; 155,9; |
| 3 | 194–195 | EtOH/$H_2O$ 1:1 | 20,0; 20,0; 27,4; 43,8; 51,0; 106,9; 117,5; 132,2; 140,5; 149,4; 151,0; 156,1; |
| 4 | 226–227 | EtOH/H2O 1:1 | 14,8; 19,9; 19,9; 27,2; 43,7; 50,8; 106,5; 117,2; 132,4; 150,1; 151,0; 152,3; 155,6; |
| 5 | 153–154 | EtOH/$H_2O$ 1:1 | 12,6; 13.8; 20,0; 22,7; 30,2; 43,7; 43,7; 106,7; 117,2; 132,5; 149,6; 150,8; 155,5; 157,4; |
| 6 | 186–187 | MeOH | 11,1; 14,8; 21,4; 43,7; 45,4; 106,6; 117,3; 132,4; 149,7; 150,7; 152,3; 155,6; |
| 7 | 171–172 | MeOH | 14,8; 22,5; 22,5; 26,1; 36,8; 42,6; 43,7; 106,7; 117,3; 132,4; 149,7; 150,6; 152,4; 165,6; |
| 8 | 214–215 | EtOH/$H_2O$ 1:1 | 13,3; 13,8; 14,7; 20,0; 30,2; 36,9; 43,7; 106,8; 149,5; 150,7; 152,1; 155,7; |
| 9 | 153–154 | EtOH/$H_2O$ 1:1 | 12,5; 13,3; 13,8; 20,0; 22,7; 30,3; 36,9; 43,7; 106,9; 149,4; 150,9; 155,7; 157,1; |
| 10 | 196–197 | MeOH | 13,8; 14,7; 20,3; 30,2; 41,7; 45,7; 106,8; 117,8; 131,6; 149,2; 150,7; 152,2; 155,8; |
| 11 | 174–175 | MeOH | 11,4; 13,8; 14,7; 20,0; 21.5; 30,2; 43,3; 43,8; 106,8; 149,5; 151,0; 152,1; 155,9; |
| 12 | 230–231 | MeOH | 11,4; 14,7; 19,9; 19,9; 21,4; 27,3; 43,3; 50,8; 106,7; 149,9; 151,2; 152,0; 155,9; |
| 13 | 189–190 | EtOH/$H_2O$ 1:1 | 11,1; 13,8; 14,7; 20,3; 21,4; 30,2; 41,6; 45,4; 106,8; 149,6; 150,9; 152,1; 155,9; |
| 14 | 202–203 | EtOH/$H_2O$ 1:1 | 11,1; 11,5; 14,7; 21,4; 21,4; 43,3; 45,4; 106,8; 149,5; 151,0; 152,1; 155,8; |
| 15 | 172–173 | EtOH/$H_2O$ 1:1 | 13,8; 14,8; 19,7; 19,7; 20,1; 30,2; 43,7; 46;4; 107,1; 149,4; 150,9; 152,0; 156,5; |

Exemple 16

Les xanthines des exemples précédents ont fait l'objet d'une étude comportementale chez le rat en fonction de l'anxiété induite par un nouvel environment. Chez le rat, l'anxiété se manifeste par des redressements de celui-ci sur ses deux pattes postérieures, tandis que ses déplacements sont l'expression de l'activité locomotrice.

Méthode

L'induction d'un état d'anxiété s'effectue par confrontation de rats mâles "naïfs" Sprague-Dawley (Iffa-Credo, France), pesant 260 à 300 g avec un nouvel environnement constitué par des cages en Macrolon$^R$ (30 × 25 cm) qui se situent dans une chambre insonorisée et climatisée (22°C et 50% d'humidité relative).

La détermination du nombre de redressements et de déplacements a lieu de façon automatique à partir de cellules photo-électriques à infrarouge qui n'émettent des impulsions électriques que pour les mouvements francs de l'animal et pas pour les mouvements statiques comme ceux de la tête et de la queue et ceci pour des raisons de reproductibilité. Ces cellules photo-électriques quadrillent les cages à deux niveaux différents de façon à distinguer les redressements des déplacements. Le nombre de redressements et de déplacements est compté par les deux séries de cellules photo-électriques, mémorisé et restitué sur une imprimante selon un programme préétabli.

On administre oralement par intubation oesophagienne le composé testé en solution sous forme de son sel de sodium, l'administration de référence étant constituée par 6 ml/kg d'eau distillée, et ceci 30 minutes avant de placer les animaux dans les cages. Le nombre de redressements et de déplacements est déterminé durant les 15 minutes qui suivent la mise en cage, par rapport à l'effet engendré par l'administration de référence.

Traitement des résultats

On détermine à partir de l'évolution des effets des substances testées sur le nombre de déplacements et de redressements en fonction des doses progressives administrées exprimé en pourcentage des déplacements et redressements comptés pour l'administration de référence, la dose ED 50 (exprimée en mg/kg) c'est-à-dire la quantité de substance nécessaire pour procurer une variation de 50% de l'activité motrice par rapport à l'administration de référence.

A partir des données précédentes, on établit les droites de régression pour les déplacements et les redressements selon la méthode décrite par Saubrié, P. J. Pharmacol, (Paris) 2 (1971) 457—472, l'équation de ces droites de régression correspondant à la moyenne exprimée en % des performances des références en fonction du produit par 10 du logarithme des doses (on ne considère que les doses ne diminuant pas l'activité motrice de plus de 75%).

La comparaison des pentes des droites de régression correspondant aux déplacements et aux redressements est effectuée en portant sur un système d'axes orthonormés en abscisse (d) la valeur de la pente de la droite des déplacements et en ordonnée (r) celle de la pente des redressements. Ces valeurs mettent en évidence la relation effet-dose pour une substance donnée.

On détermine également le rapport de la pente des déplacements sur celle des redressements qui permet de juger de la spécificité d'effet d'une substance donnée sur l'anxiété.

Le tableau 3 ci-après montre les résultats obtenus:

TABLEAU 3

| Composé de l'exemple | ED 50 (mg/kg) | Pente des déplacements ≠ 0 (d) | Pente des redressements ≠ 0 (r) | Rapport des pentes d/r | Effet |
|---|---|---|---|---|---|
| 1 | 0,14 | P <0,001 | P <0,01 | n.s. | S.N. |
| 2 | 1,60 | P <0,001 | P <0,001 | <1; P<0,001 | S.A. |
| 3 | 0,98 | P <0,001 | P <0,001 | <1; P <0,001 | S.A. |
| 4 | 0,25 | P <0,001 | P <0,01 | n.s. | S.N. |
| 5 | 6,24 | P <0,01 | P <0,001 | <1; P <0.001 | S.A. |
| 6 | 0,27 | P <0,001 | P <0,001 | <1; P <0,01 | S.A. |
| 7 | 1,03 | P <0,001 | P <0,01 | <1; P <0,01 | S.A. |
| 8 | 1,02 | P <0.05 | P <0,01 | <1; P <0,05 | S.A. |
| 9 | 2,38 | P < 0,001 | P <0,001 | <1; P <0,001 | S.A. |
| 10 | 3,95 | P <0,01 | P <0,01 | n.s. | S.N. |
| 11 | 0,35 | P <0,001 | P <0,001 | <1; P <0,001 | S.A. |
| 12 | 0,25 | P <0,01 | P <0,01 | <1; P <0,001 | S.A. |
| 13 | 1,05 | P <0,01 | P <0,01 | <1; P <0,05 | S.A. |
| 14 | 0,37 | P <0,01 | P <0,001 | <1; P <0,01 | S.A. |
| 15 | 1,76 | P <0,05 | P <0,01 | n.s. | S.N. |
| chlorpromazine | 21 | P <0,05 | P <0,05 | n.s. | S.N. |
| halopéridol | 1 | P <0,01 | P <0,01 | n.s. | S.N. |
| chlordiazépoxide | 12 | P <0,01 | P <0,01 | <1; P <0,01 | S.A. |
| théophyline | – | P <0,001 | n.s. | >1; P <0,001 | P |

Légende

La comparaison des pentes est effectuée à l'aide du test "t" aux probabilités $P<0,05$, $P<0,01$ et $P<0,001$.

n.s. signifie que la pente est non significativement différente de 0 ou que le rapport entre les pentes des déplacements et des redressements est non significativement différent de 1.

S.N. signifie que le composé a un effet sédatif non spécifique.

S.A. indique un effet sédatif à caractère anxiolytique.

P vent dire psychostimulant.

Conclusions

La détermination de l'effet d'une substance est donnée par sa localisation par rapport à la droite d = r de pente = 1. Les substances situées sur cette droite ont un effet semblable sur les redressements et les déplacements, ce sont des sédatifs non spécifiques (S.N). Pour ces composés, la valeur de ED 50 indiquée correspond à la moyenne tirée des deux droites de régression.

Les substances pour lesquelles la valeur absolue de la pente des redressements est significativement supérieure à celle des déplacements ont une action spécifique sur l'anxiété. Ce sont des sédatifs à caractère anxiolytique (S.A.). Dans de dernier cas, la ED 50 est calculée à partir de la droite de régression des redressements.

Le tableau 3 ci-dessus montre:

que les composés des exemples 1, 4, 10 et 15 ont un profil d'action semblable aux neuroleptiques chlorpromazine et halopéridol,

que les composés des exemples 2, 3, 5 à 9 et 11 à 14 sont un profil d'action semblable au chlordiazépoxide,

que la théophylline est psychostimulante et n'a pas d'effet sur l'anxiété.

Exemple 17

Les composés des exemples 1, 2, 4, 11 et 12 ont été soumis à des tests toxicologiques:

a) Détermination de la toxicité aiguë LD 50 chez la souris mâle et femelle en solution dans le DMSO, la référence étant constituée du DMSO seul selon J. T. Litchfield et F. Wilcoxon, "Simplified method of evaluating dose-effect experiments", Journal of pharmacology and experimental therapy vol. 96, p. 99—113, 1949.

b) Détermination de la toxicité aiguë LD 50 chez le rat mâle et femelle en solution dans le DMSO (référence DMSO seul) selon C. S. Veil et G. J. Wright, "Intra- and inter laboratory comparative evaluation of single oral test", Toxicology and applied pharmacology. Vol. 11, p. 378—388, 1967.

Les composés des exemples 1, 2, 3, 4, 5, 9 et 11 ont montré dans un screening de base les activités:

c) Diurétique chez le rat mâle selon W. L. Lipschitz, Z. Hadidian et A. Kerpcsar, JPET, Vol. 79, p. 97—110, 1943.

d) Antiallergique chez le rat mâle et femelle selon J. Goose et A. M. J. N. Blair, Immunol., Vol. 16, p. 749—760, 1969.

e) Bronchodilatatrice in vitro selon F. P. Luduena et al, Arch, int. pharmacodyn., Vol. 111, p. 392—400, 1957.

f) Antihistaminique in vitro selon R. Magnus, Arch. f.d., ges. physiol., Vol. 102, p. 123—151, 1904.

Les résultats de ces tests sont consignés dans le tableau 4 ci-dessous:

TABLEAU 4

| Composé de l'exemple | a LD 50 en mg/kg p.o., souris mâle | a LD 50 en mg/kg p.o., souris femelle | b LD 50 en mg/kg p.o., rat mâle | b LD 50 en mg/kg p.o., rat femelle | c dose minimale efficace en mg/kg produisant une résponse significative | d dose minimale efficace en mg/kg produisant une résponse significative | e concentration minimale efficace en $\mu g/ml$ produisant une résponse significative | f concentration minimale efficace en $\mu g/ml$ produisant une résponse significative |
|---|---|---|---|---|---|---|---|---|
| 1 | 72 | 62 | 35 | 24 | 5 | 2,5 | 100 | 100 |
| 2 | – | – | >200 | >200 | 10 | 10 | 25 | 100 |
| 3 | – | – | – | – | 5 | sans effet | sans effet | 200 |
| 4 | 79 | 49 | 23 | 17 | 20 | 50 | sans effet | sans effet |
| 5 | – | – | – | – | 2,5 | sans effet | 5 | 100 |
| 9 | – | – | – | – | 5 | sans effet | 25 | 50 |
| 11 | 91 | 151 | 25 | 15 | 0,25 | sans effet | 10 | 50 |
| 12 | – | – | 28 | 19 | – | – | – | – |

Légende: – signifie non testé.

Les composés des exemples 1, 4, 11 et 12 ont été soumis à des tests comportementaux selon "Predictability and specificity of behaviour screening tests for neuroleptic", P. Worms et K. G. Lloyd, Pharmacology, Teratology, Vol. 5, p. 445—450, 1979, comprenant:

catalepsie induite par l'halopéridol chez le rat: potentialisation à 1 mg/kg p.o.

analgésie induite par le pentobarbital chez la souris: potentialisation à 0,5 mg/kg p.o.

"climbing behaviour" induit par l'apomorphine chez la souris: inhibition à 1 mg/kg p.o.

comportement stéréotypé induit par l'apomorphine chez le rat: pas d'antagonisme jusqu'à 8 mg/kg p.o.

température rectale chez la souris: effet hypothermisant à 2 mg/kg p.o.

hypermotilité induite par l'amphétamine chez le rat: antagonisme à 0,5 mg/kg p.o.

Ces effets sont typiques des neuroleptiques sauf qu'à l'inverse des neuroleptiques comme l'halopéridol ou la chlorpromazine, les composés ci-dessus sont sans effet sur le comportement stéréotypé chez le rat, induit par l'apomorphine.

**Revendications**

1. Composé de formule

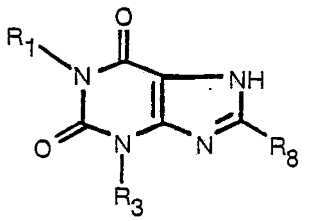

(I)

ou un sel physiologiquement acceptable de ce composé, formule dans laquelle $R_1$ est alkyle en $C_2$—$C_4$, isoalkyle en $C_3$—$C_4$, $CH_2$-(alcényle en $C_2$—$C_3$) ou $CH_2$-(isoalcényle en $C_3$), $R_3$ est alkyle en $C_3$—$C_5$, isoalkyle en $C_3$—$C_5$, $CH_2$-(alcényle en $C_2$—$C_4$) ou $CH_2$-(isoalcényle en $C_3$—$C_4$), $R_8$ est H, méthyle ou éthyle, sauf que 1) lorsque $R_8$ est H, $R_1$ est allyle, 2) $R_1$ et $R_3$ ne sont pas simultanément butyle, allyle ou isobutyle et 3) lorsque $R_1$ est éthyle, $R_3$ est différent de 2-méthylbutyle.

2. Composé selon la revendication 1, dans lequel $R_8$ est méthyle ou un sel physiologiquement acceptable de celui-ci.

3. Composé selon la revendication 1, dans lequel $R_1$ est allyle, $R_3$ est butyle ou isobutyle et $R_8$ est H ou un sel physiologiquement acceptable de celui-ci.

4. Composé selon la revendication 2, dans lequel $R_1$ est allyle, propyle ou isobutyle et $R_3$ est propyle, butyle ou isobutyle ou un sel physiologiquement acceptable de celui-ci.

5. 1-Allyl-3-butyl-8-méthylxanthine selon la revendication 4 ou un sel physiologiquement acceptable de ce composé.

6. 1-Propyl-3-butyl-8-méthylxanthine selon la revendication 4 ou un sel physiologiquement acceptable de ce composé.

7. 1-Allyl-3-isobutyl-8-méthylxanthine selon la revendication 4 ou un sel physiologiquement acceptable de ce composé.

8. 1-Propyl-3-isobutyl-8-méthylxanthine selon la revendication 4 ou un sel physiologiquement acceptable de ce composé.

9. 1,3-Dipropyl-8-méthylxanthine selon la revendication 4 ou un sel physiologiquement acceptable de ce composé.

10. 1-Allyl-3-propyl-8-méthylxanthine selon la revendication 4 ou un sel physiologiquement acceptable de ce composé.

11. Procédé de préparation d'un composé de formule

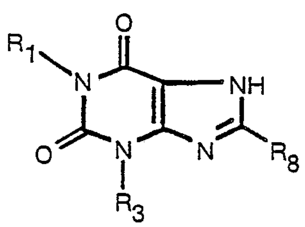

(I)

ou un sel physiologiquement acceptable de ce composé, formule dans laquelle $R_1$ est alkyle en $C_2$—$C_4$, isoalkyle en $C_3$—$C_4$, $CH_2$-(alcényle en $C_2$—$C_3$) ou $CH_2$-(isoalcényle en $C_3$), $R_3$ est alkyle en $C_2$—$C_5$, isoalkyle en $C_3$—$C_5$, $CH_2$-(alcényle en $C_2$—$C_4$) ou $CH_2$-(isoalcényle en $C_3$—$C_4$), $R_8$ est H, méthyle ou éthyle, sauf que 1) lorsque $R_8$ est H, $R_1$ est allyle, 2) $R_1$ et $R_3$ ne sont pas simultanément butyle, allyle ou isobutyle et 3) lorsque $R_1$ est éthyle, $R_3$ est différent de 2-méthylbutyle, caractérisé par le fait que

a) on fait réagir un composé de formule

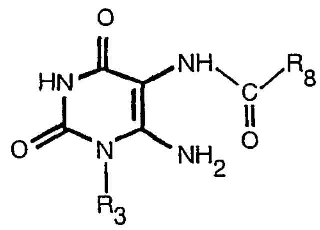

avec un agent alkylant de formule $R_1$—X où $R_1$, $R_3$ et $R_3$ sont définis comme ci-dessus sauf que $R_1$ et $R_3$ sont différents de isopropyle et X est un halogène ou mono- ou di-sulfate ou p-toluènesulfonate et on cyclise le composé obtenu ou

b) on hydrogène un composé obtenu selon a) dans lequel au moins l'un de $R_1$ et $R_3$ est alcényle ou isoalcényle tel que défini ci-dessus ou

c) on fait réagir un composé de formule

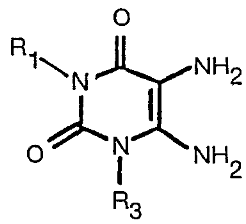

avec un acide de formule $R_8$—COOH où $R_1$, $R_3$ et $R_8$ sont définis comme ci-dessus sauf que $R_1$ et $R_3$ sont différents de isopropyle et on cyclise le composé obtenu ou

d) on fait réagir un composé de formule

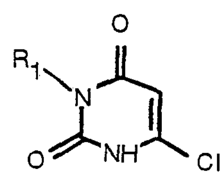

avec un agent alkylant de formule $R_3$—X puis on traite le composé obtenu par une amine de formule $R_8$—$CH_2$—$NH_2$ où $R_1$, $R_3$, X et $R_8$ sont définis comme ci-dessus de façon à obtenir un composé de formule

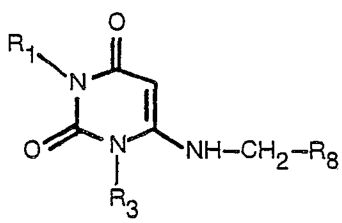

qu'on transforme en dérivé 5-nitroso de formule

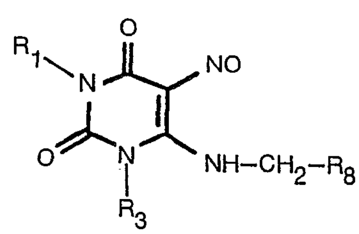

qui cyclise spontanément ou qu'on cyclise par chauffage, et on convertit, si on le désire, le composé obtenu par l'une des voies a)—d) en un de ses sels physiologiquement acceptable.

12. Composition pharmaceutique, caractérisée en ce qu'elle contient un composé de formule

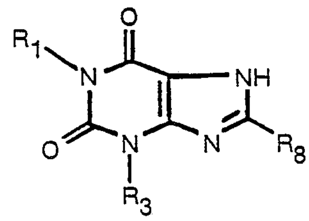

(I)

ou un sel physiologiquement acceptable de ce composé, formule dans laquelle $R_1$ est alkyle en $C_2$—$C_4$, isoalkyle en $C_3$—$C_4$, $CH_2$-(alcényle en $C_2$—$C_3$) ou $CH_2$-(isoalcényle en $C_3$), $R_3$ est alkyle en $C_3$—$C_5$, isoalkyle en $C_3$—$C_5$, $CH_2$-(alcényle en $C_2$—$C_4$) ou $CH_2$-(isoalcényle en $C_3$—$C_4$), $R_8$ est H, méthyle ou éthyle, sauf que 1) lorsque $R_8$ est H, $R_1$ est allyle, et 2) $R_1$ et $R_3$ ne sont pas simultanément butyle ou allyle et 3) lorsque $R_1$ est éthyle, $R_3$ est différent de 2-méthylbutyle en liaison avec un excipient pharmaceutiquement acceptable.

13. Composition selon la revendication 12, caractérisée en ce qu'elle contient un composé selon l'une des revendications 2 à 10.

**Patentansprüche**

1. Verbindung der Formel

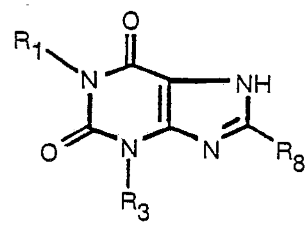

(I)

oder ein physiologisch annehmbares Salz dieser Verbindung, wobei in der Formel sind: $R_1$ Alkyl mit $C_2$—$C_4$, Isoalkyl mit $C_3$—$C_4$, $CH_2$-(Alkenyl mit $C_2$—$C_3$) oder $CH_2$-(Isoalkenyl mit $C_3$), $R_3$ Alkyl mit $C_3$—$C_5$, Isoalkyl mit $C_3$—$C_5$, $CH_2$-(Alkenyl mit $C_2$—$C_4$) oder $CH_2$-(Isoalkenyl mit $C_3$—$C_4$), $R_8$ Wasserstoff, Methyl oder Ethyl, mit der Maßgabe, Daß 1) wenn $R_8$ Wasserstoff ist, $R_1$ Alkyl ist, 2) $R_1$ und $R_3$ nicht gleichzeitig Butyl, Allyl oder Isobutyl sind und 3) wenn $R_1$ Ethyl ist, $R_3$ ein anderer Rest als 2-Methylbutyl ist.

2. Verbindung nach Anspruch 1, in welcher $R_8$ Methyl ist oder ein physiologisch annehmbares Salz davon.

3. Verbindung nach Anspruch 1, bei der $R_1$ Allyl ist, $R_3$ Butyl oder Isobutyl ist und $R_8$ Wasserstoff ist oder ein physiologisch annehmbares Salz davon.

4. Verbindung nach Anspruch 2, bei der $R_1$ Allyl, Propyl oder Isobutyl ist und $R_3$ Propyl, Butyl oder Isobutyl ist oder ein physiologisch annehmbares Salz davon.

5. 1-Allyl-3-butyl-8-methylxanthin nach Anspruch 4 oder ein physiologsich annehmbares Salz dieser Verbindung.

6. 1-Propyl-3-butyl-8-methylxanthin nach Anspruch 4 oder ein physiologisch annehmbares Salz dieser Verbindung.

7. 1-Allyl-3-isobutyl-8-methylxanthin nach Anspruch 4 oder ein physiologisch annehmbares Salz dieser Verbindung.

8. 1-Propyl-3-isobutyl-8-methylxanthin nach Anspruch 4 oder ein physiologisch annehmbares Salz dieser Verbindung.

9. 1,3-Dipropyl-8-methylxanthin nach Anspruch 4 oder ein physiologisch annehmbares Salz dieser Verbindung.

10. 1-Allyl-3-propyl-8-methylxanthin nach Anspruch 4 oder ein physiologisch annehmbares Salz dieser Verbindung.

11. Verfahren zur Herstellung einer Verbindung der Formel

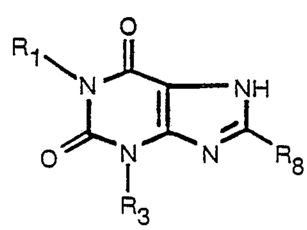

(I)

oder eines physiologisch annehmbaren Salzes dieser Verbindung, wobei in der Formel sind: $R_1$ Alkyl mit $C_2$—$C_4$, Isoalkyl mit $C_3$—$C_4$, $CH_2$-(Alkenyl mit $C_2$—$C_3$) oder $CH_2$-(Isoalkenyl mit $C_3$), $R_3$ Alkyl mit $C_3$—$C_5$,

Isoalkyl mit $C_3$—$C_5$, $CH_2$-(Alkenyl mit $C_2$—$C_4$) oder $CH_2$-(Isoalkenyl mit $C_3$—$C_4$), $R_8$ Wasserstoff, Methyl oder Ethyl, mit der Maßgabe, daß 1) wenn $R_8$ H ist, $R_1$ Allyl ist, 2) $R_1$ und $R_3$ nicht gleichzeitig Butyl, Allyl oder Isobutyl sind und 3) wenn $R_1$ Ethyl ist, $R_3$ ein anderer Rest als 2-Methylbutyl ist, dadurch gekennzeichnet, daß man

a) eine Verbindung der Formel

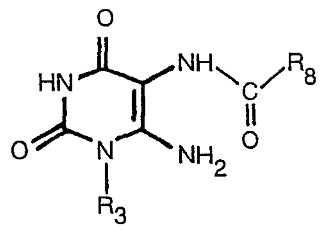

mit einem Alkylierungsmittel der Formel $R_1$—X umsetzt, wobei $R_1$, $R_3$ und $R_8$ wie oben definiert sing, mit der Maßgabe, daß $R_1$ und $R_3$ andere Reste als Isopropyl sing, und wobei X Halogen oder Mono- oder Disulfat oder p-Toluolsulfonat ist, und die erhaltene Verbindung cyclisiert oder

b) eine Verbindung hydriert, die gemäß a) erhalten wurde, bei der wenigstens einer der Reste $R_1$ und $R_3$ Alkenyl oder Isoalkenyl ist, wie sie oben definiert wurden, oder

c) eine Verbindung der Formel

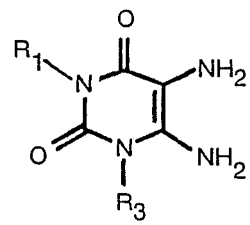

mit einer Säure der Formel $R_8$—COOH umsetzt, wobei $R_1$, $R_3$ und $R_8$ wie oben definiert sind, mit der Maßgabe, daß $R_1$ und $R_3$ andere Reste als Isopropyl sind, und die erhaltene Verbindung cyclisiert oder

d) eine Verbindung der Formel

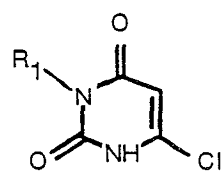

mit einem Alkylierungsmittel der Formel $R_3$—X umsetzt, dann die erhaltene Verbindung mit einem Amin der Formel $R_8$—$CH_2$— $NH_2$ behandelt, wobei $R_1$, $R_3$, X und $R_8$ wie oben definiert sind, und zwar in einer Weise, daß eine Verbindung der Formel erhalten wird

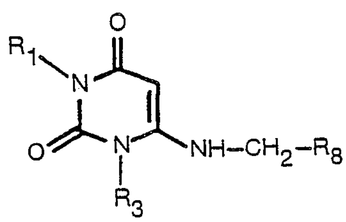

die man in ein 5-Nitrosoderivat der Formel überführt

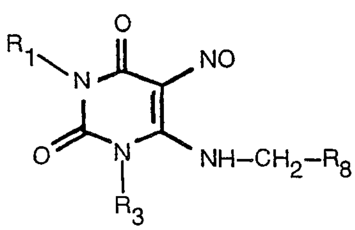

die spontan cyclisiert oder die man unter Erwärmen cyclisiert, und, wenn gewünscht, die nach einem der Wege a) bis d) erhaltene Verbindung in eines ihrer physiologisch annehmbaren Salze überführt.

12. Pharmazeutische Zusammensetzung, dadurch gekennzeichnet, daß sie eine Verbindung der Formel enthält

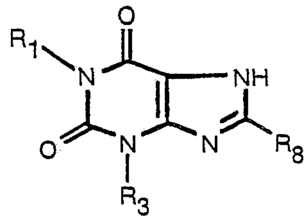

(I)

oder ein physiologisch annehmbares Salz dieser Verbindung, wobei in der Formel sind: $R_1$ Alkyl mit $C_2$—$C_4$, Isoalkyl mit $C_3$—$C_4$, $CH_2$-(Alkenyl mit $C_2$—$C_3$) oder $CH_2$-(Isoalkenyl mit $C_3$), $R_3$ Alkyl mit $C_3$—$C_5$, Isoalkyl mit $C_3$—$C_5$, $CH_2$-(Alkenyl mit $C_2$—$C_4$) oder $CH_2$-(Isoalkenyl mit $C_3$—$C_4$), $R_8$ H, Methyl oder Ethyl, mit der Maßgabe, daß 1) wenn $R_8$ Wasserstoff ist, $R_1$ Allyl ist, und 2) $R_1$ und $R_3$ nicht gleichzeitig Butyl oder Allyl sind, und 3) wenn $R_1$ Ethyl ist, $R_3$ ein anderer Rest als 2-Methylbutyl ist, in Verbindung mit einem pharmazeutisch annehmbaren Hilfsstoff.

13. Zusammensetzung nach Anspruch 12, dadurch gekennzeichnet, daß sie eine Verbindung nach einem der Ansprüche 2 bis 10 enthält.

**Claims**

1. Compounds corresponding to the formula

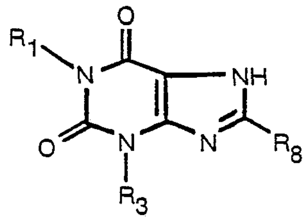

(I)

or a physiologically acceptable salt thereof in which $R_1$ represents $C_2$—$C_4$-alkyl, $C_3$—$C_4$-isoalkyl, $CH_2$—($C_2$—$C_3$-alkenyl or $CH_2$—($C_3$-isoalkenyl); $R_3$ represents $C_3$—$C_5$-alkyl, $C_3$—$C_5$-isoalkyl, $CH_2$—($C_2$—$C_4$-alkenyl) or $CH_2$—($C_3$—$C_4$-isoalkenyl); $R_8$ represents H, methyl or ethyl; with the proviso that 1) when $R_8$ represents H, $R_1$ is allyl, 2) $R_1$ and $R_3$ cannot both represent butyl, allyl or isobutyl at the same time and 3) when $R_1$ is ethyl, $R_3$ is different from 2-methylbutyl.

2. Compounds as claimed in Claim 1 wherein $R_8$ is methyl or a physiologically acceptable salt thereof.

3. Compounds as claimed in Claim 1 wherein $R_1$ is allyl, $R_3$ is butyl or isobutyl and $R_8$ is H or a physiologically acceptable salt thereof.

4. Compounds as claimed in Claim 2 wherein $R_1$ is allyl, propyl or isobutyl and $R_3$ is propyl, butyl or isobutyl or a physiologically acceptable salt thereof.

5. 1-Allyl-3-butyl-8-methyl xanthine or a physiologically acceptable salt thereof.

6. 1-Propyl-3-butyl-8-methyl xanthine or a physiologically acceptable salt thereof.

7. 1-Allyl-3-isobutyl-8-methyl xanthine or a physiologically acceptable salt thereof.

8. 1-Propyl-3-isobutyl-8-methyl xanthine or a physiologically acceptable salt thereof.

9. 1,3-Dipropyl-8-methyl xanthine or a physiologically acceptable salt thereof.

10. 1-Allyl-3-propyl-8-methyl xanthine or a physiologically acceptable salt thereof.

11. A process for the preparation of compounds corresponding to the formula

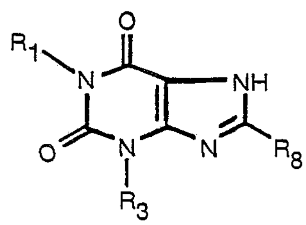

(I)

or a physiologically acceptable salt thereof in which $R_1$ represents $C_2$—$C_4$-alkyl, $C_3$—$C_4$-isoalkyl, $CH_2$-($C_2$—$C_3$-alkenyl) or $CH_2$-($C_3$-isoalkenyl); $R_3$ represents $C_3$—$C_5$-alkyl, $C_3$—$C_5$-isoalkyl, $CH_2$—($C_2$—$C_4$-alkenyl) or $CH_2$—($C_3$—$C_4$-isoalkenyl); $R_8$ represents H, methyl or ethyl; with the proviso tha1 1) when $R_8$ represents H,

$R_1$ is allyl, 2) $R_1$ and $R_3$ cannot both be butyl, allyl or isobutyl at the same time and 3) when $R_1$ is ethyl, $R_3$ is different from 2-methylbutyl, which comprises

a) reacting a compound corresponding to the following formula

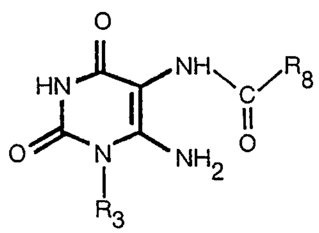

with an alkylating agent of the formula $R_1$—X where $R_1$, $R_3$ and $R_8$ are as defined above except that $R_1$ and $R_3$ are other than isopropyl and X is a halogen or monosulphate or disulphate of p-toluene sulphonate and cyclising the compound obtained, or;

b) hydrogenating a compound of formula (I) in which at least one of $R_1$ and $R_3$ is alkenyl or isoalkenyl, as defined above, or;

c) reacting a compound corresponding to the formula

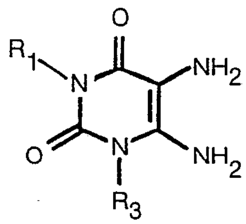

with an acid of the formula $R_8$—COOH where $R_1$, $R_3$ and $R_8$ are as defined above, except that $R_1$ and $R_3$ are other than isopropyl, and cyclising the compound obtained; or

d) reacting a compound corresponding to the formula

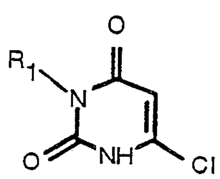

with an alkylating agent of the formula $R_3$—X, treating the compound obtained with an amine of the formula $R_8$—$CH_2$—$NH_2$ where $R_1$, $R_3$, X and $R_8$ are as defined above, to form a compound corresponding to the following formula

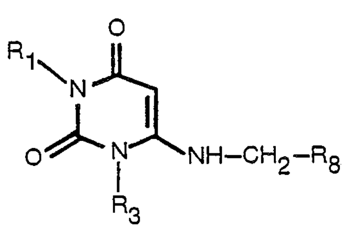

which is converted into a 5-nitroso derivative corresponding to the formula

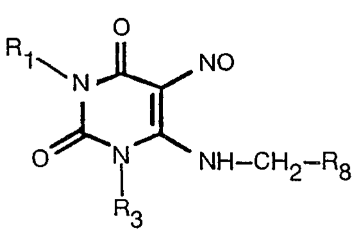

which cyclises spontaneously or which is cyclised by heating; and, if desired, converting the compound obtained into a physiologically acceptable salt.

12. A pharmaceutical composition, comprising a compound of the formula

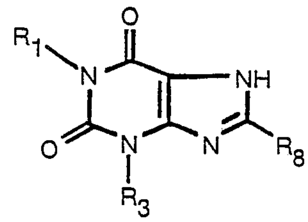

(I)

or a physiologically acceptable salt thereof in which $R_1$ represents $C_2$—$C_4$-alkyl, $C_3$—$C_4$-isoalkyl, $CH_2$—($C_2$—$C_3$-alkenyl) or $CH_2$—($C_3$-isoalkenyl); $R_3$ represents $C_3$—$C_5$-alkyl, $C_3$—$C_5$-isoalkyl, $CH_2$—($C_2$—$C_4$-alkenyl) or $CH_2$—($C_3$—$C_4$-isoalkenyl); $R_8$ represents H, methyl or ethyl; with the proviso that 1) when $R_8$ represents H, $R_1$ is allyl, 2) $R_1$ and $R_3$ cannot both represent butyl or allyl and 3) when $R_1$ is ethyl, $R_3$ is different from 2-methylbutyl together with an inert carrier or diluent pharmaceutically acceptable.

13. A composition as claimed in claim 12 comprising a compound as claimed in any of claims 2 to 10.